# EUROPEAN PATENT APPLICATION

(11) **EP 3 680 667 A1**
(43) Date of publication of application: **15.07.2020**
(21) Application number: 19151227.6
(22) Date of filing: 10.01.2019
(51) Int. Cl.: G01N 33/68, C07K 5/09, G01N 33/50

(54) **HIGHLY SELECTIVE AND ULTRASENSITIVE CU2+ ION SENSOR**

(71) Applicant: Tietze, Alesia, 43145 Mölndal (SE)
(72) Inventor: TIETZE, Alesia Dr., 43145 Mölndal (SE)
(74) Representative: Weickmann & Weickmann PartmbB

(57) **Abstract**

The present application is directed to a sensor for detection of Cu²⁺ ions in a fluid comprising a copper-binding motif 2,3-diaminopropionic acid(DAP)-βAla-X, wherein X is an amino acid or a modified amino acid, in particular wherein X is histidine (His), alanine (Ala) or aspartic acid (Asp). The sensor according to the present application can be used for fast, highly selective and ultrasensitive detection of Cu²⁺ ions in a fluid. With said sensor a qualitative and/or quantitative detection of Cu²⁺ ions can be achieved, which can be useful in the diagnosis and/or monitoring of diseases linked to abnormal Cu²⁺ ion concentrations and/or dysregulated copper metabolism, in particular Alzheimer's disease.

## Description

The present application is directed to the use of a copper-binding motif for binding Cu²⁺ ions in a fluid, a sensor for detection of Cu²⁺ ions in a fluid, the use of the sensor in the diagnosis and monitoring of diseases and a method for qualitative and quantitative detection of Cu²⁺ ions in a fluid sample.

Copper is an essential trace element that is inevitable in biological systems and can be found in many enzymes such as amine oxidases and ferroxidases but is also required for infant growth, the ion metabolism and brain development in human organisms (Nat Chem Biol 2008, 4, 176-185; J Toxicol Environ Health B Crit Rev 2001, 4, 341-394). Copper is also widely used in agricultural systems and therefore belongs to a major metal pollutant in our environment (Rev Environ Health 1999, 14, 231-238). The contamination of ecosystems such as rivers, lakes, soil and groundwater with copper causes increased accumulation (Chem Soc Rev 2017, 46, 7105-7123).

There are also diseases linked to an abnormal copper concentration and/or dysregulated copper metabolism in humans such as Wilson's disease, Menke's disease and Alzheimer's disease (AD) (Water, Air and Soil Pollution 1998, 108, 457-471). Dysregulation of copper homeostasis causes neurodegenerative diseases, which led to a rising interest in the investigation of the connection between copper and AD (Dalton Trans 2004, 1907-1917). The involvement of copper in AD contains two aspects: first, aggregation/plaque formation of so-called β-amyloid peptide (Aβ), which is cleaved from amyloid precursor protein (APP) upon copper binding and second, oxidative stress which is caused by reduction of Cu²⁺ to Cu¹⁺ through its binding domain (CuBD) (Dalton Trans 2004, 1907-1917; Neuron 2001, 30, 665-676). Both aspects are known to aggravate AD which results in cell and especially neuronal death (J Toxicol Environ Health B Crit Rev 2001, 4, 341-394).

During the last two decades studies have been performed in order to confirm the involvement of copper in β-amyloid plaque progression. Schreurs et al. (Proc Natl Acad Sci USA 2003, 100, 11065-11069) observed an increased formation of β-amyloid plaques by cholesterol-fed rabbits when adding trace amounts (12 ppm) of copper to their drinking water. During meta-analysis performed by Squitti et al. (J Alzheimers Dis 2014, 38, 809-822) the concentration of free copper in the serum of AD patients was determined to be higher compared to that of healthy individuals. These results were generated by studying the fraction of copper that is not bound to the ceruloplasmin (non-CP Cu), i.e. free Cu²⁺ in solution.

Recent studies of Squitti et al. (J Trace Elem Med Biol 2018, 45, 181-188) show the potential of non-CP Cu detection, i.e. detection of free Cu²⁺ in urine as an eligible marker for patients in early stages of AD. In particular, Squitti et al. report a 2.4 times increased concentration of copper in urine of AD patients in comparison to healthy controls. Thus, determination of non-bound Cu²⁺ in urine and serum can be used as a basis for a screening method for the early diagnosis and/or monitoring of diseases linked to abnormal copper concentrations, for example AD or Wilson's disease.

In recent years first steps have been taken towards the development of screening methods and sensors for the determination of copper for early AD diagnostics. For example, Hirayama et al. (Proc Nat Acad Sci USA 2012, 109, 2228-2233) developed a copper-imaging sensor inside of living cells with a sensitive fluorescence turn-on response to Cu¹⁺. However, this method depends on live-cell imaging after digestion of the fluorescent dye and thus the detection method itself and its implementation is tied to an extensive set-up. Squitti et al. (J Alzheimers Dis 2014, 38, 809-822) designed a method using fluorescence to detect non-CP Cu²⁺ distributed in the serum. However, also this method requires an extensive two-step experimental set-up with a size exclusion solid-phase extraction separating non-CP Cu from protein-bound copper followed by a fluorescent method, which also requires a lengthy time period to obtain results.

It is known that peptides in proteins can display extraordinary and ultrasensitive key-lock behavior towards targets. Copper-binding peptides in nature are albumin (bovine serum albumin (BSA), human serum albumin (HSA), rabbit serum albumin (RSA)), neuromedin C and K, human sperm protamine P2a and histidines (Accounts of Chemical Research 1997, 30, 123-130). Imperiali et al. (J Am Chem Soc 1998, 120, 609-610) reported polypeptide motifs for the design of selective Cu(ll) ion chemosensors. Papp et al. (Angewandte Chem Int Ed Engl 2018, 57, 4752-4755) were able to attach the amino terminal Cu(II) and Ni(II)-binding (ATCUN) motif (Gly-Gly-His) on track-etched polycarbonate membranes. A copper-sensing system that can be evaluated by naked eye detection with a detection limit of 0.5 µM using a "turn-on" fluorescence strategy is reported by Situ et al. (Sensors and Actuators B-Chemical 2017, 240, 560-565), however, with the disadvantage that the detection method involves several reaction steps. Further, naked eye detectable sensor systems were constructed by Ding et al. (Journal of Materials Chemistry 2011, 21, 13345-13353) presenting a colorimetric detection of copper using sensor strips at a detection limit of 5 nM.

Due to the above-outlined disadvantages of the known copper sensing methods and the need for highly sensitive and selective detection methods for Cu²⁺ ions it is an object of the present application to provide a sensor allowing a fast and easy-to-handle, but highly sensitive and selective qualitative and quantitative detection of Cu²⁺ in a fluid, in particular for detecting elevated copper concentrations in body fluids in a quick test and in environmental samples on-site.

According to the invention, this problem has been solved by providing a sensor for detection of Cu²⁺ ions in a fluid comprising a copper-binding motif 2,3-diaminopropionic acid(DAP)-βAla-X, wherein X is an amino acid or a modified amino acid, in particular wherein X is histidine (His), alanine (Ala) or aspartic acid (Asp). The sensor according to the present application can be used for fast, highly selective and ultrasensitive detection of Cu²⁺ ions in a fluid. With said sensor a qualitative and/or quantitative detection of Cu²⁺ ions can be obtained, for example by measuring the change in fluorescence intensity, by measuring the change in the current-voltage characteristics and/or by subjecting the sensor to mass spectrometry.

The detection method of the present invention does not require an extensive experimental set-up. In fact, with the sensor of the present application detection of Cu²⁺ ions is possible on-site and in real time. For example, simply a test strip comprising the sensor having the particular copper-binding motif and a fluorescence-light source may be required. In addition or alternatively, the qualitative and quantitative detection of Cu²⁺ ions may also be performed by observing a change in the current-voltage characteristics, which even allows the detection of Cu²⁺ ion concentrations in a fluid in the femtomolar range, i.e. as low as 1 fM. M refers to the molar concentration mol per liter (mol/L)

Generally, sensors are devices for detecting particular analytes, in this case the analyte are Cu²⁺ ions. The sensor provides the information regarding the analyte usually in the form of a measurable physical signal correlated with the presence and/or concentration of the analyte.

The sensor of the present application comprises a copper-binding motif having at least two non-proteinogenic amino acids, namely 2,3-diaminopropionic acid (DAP) and β-alanine (βAla). The copper-binding motif further comprises an amino acid or modified amino acid X. The copper-binding motif corresponds to a peptide compound and therefore the copper-binding motif is sometimes termed peptide copper-binding motif. The amino acid X may be selected from the group consisting of standard amino acids, unusual amino acids or non-proteinogenic amino acids. Examples for standard L-amino acids are glycine (Gly), alanine (Ala), valine (Val), leucine (Leu), methionine (Met), isoleucine (Ile), serine (Ser), threonine (Thr), cysteine (Cys), proline (Pro), asparagine (Asn), glutamine (Gin), phenylalanine (Phe), tyrosine (Tyr), tryptophan (Trp), lysine (Lys), arginine (Arg), histidine (His), aspartic acid (Asp) and glutamate (Glu). Preferred standard amino acids according to the present invention are histidine (His), alanine (Ala) or aspartic acid (Asp). Examples for unusual amino acids are 4-hydroxyproline, 5-hydroxylysine, 6-N-methyllysine, γ-carboxyglutamate, desmosine, selenocysteine, ornithine and citroline. Non-proteinogenic amino acids are amino acids which are not incorporated in proteins during translation. Non-proteinogenic amino acids according to the present application are for example 2,3-diaminopropionic acid (DAP) and β-alanine. 2,3-diaminopropionic acid (DAP) may be L-2,3-diaminopropionic acid, D-2,3-diaminopropionic acid or a mixtures of L- and D-DAP. L-2,3-diaminopropionic acid is preferred. A modified amino acid comprises standard or unusual amino acids, which may be for example methylated, acetylated or its diastereomeric D, cis/trans and isosteric form. X is preferably a standard amino acid, more preferably histidine (His), alanine (Ala) or aspartic acid (Asp).

In a preferred embodiment the sensor of the present application comprises the copper-binding motif DAP-βAla-His or DAP-βAla-Asp.

In addition to the copper-binding motif or peptide copper-binding motif the sensor according to the present application may comprise means for detection. However, such means for detection are not mandatory since some detection methods such as mass spectrometry do not require such means for detection. Detection means or means for detection are able to produce a measurable, physical signal. In case no additional detection means are present, the N-terminus of the DAP may be capped with an acetyl (-CCH₃=O) moiety.

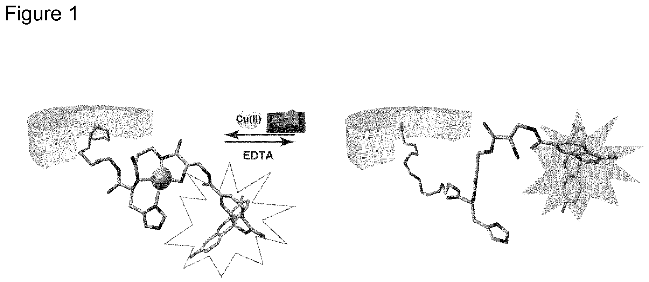

For example, the detection means according to the present application may be commercially available fluorophores such as 5(6)-carboxyfluorescein, DyLight Fluor, Alexa Fluor or cyanine dyes. A preferred fluorophore of the sensor of the present application is 5(6)-carboxyfluorescein. The fluorophore may be attached to the copper-binding motif via the N-terminus of the DAP. The quenching or amount of quenching of the fluorescence intensity indicates the presence of Cu²⁺ ions in the fluid, i.e. binding of Cu²⁺ ions to the copper-binding motif of the sensor and can be used to calculate the Cu²⁺ ions concentration in the fluid as the amount of quenching is proportional to the Cu²⁺ ion concentration in the fluid. The "on-off" characteristics of such a detection method using the sensor of the present application is shown in Figure 1.

In a preferred embodiment of the present application, the sensor comprises 5(6)-carboxyfluorescein-DAP-βAla-His or 5(6)-carboxyfluorescein-DAP-βAla-Asp.

The detection means may also comprise a polymer membrane, foil or sheet including nanopores, preferably a functionalized polymer membrane, foil or sheet with nanopores, in particular an ion-track etched polymer membrane, foil or sheet with nanopores, even more preferably an ion-track etched polyethylene terephthalate (PET)-membrane, foil or sheet with nanopores. The nanopores may have a conical shape, preferably with small openings of about 30 ± 5 nm and big openings of 500 ± 10 nm in diameter. The polymer membrane, foil or sheet preferably contains about 10⁴ nanopores per cm². Ion-track etched polymer membranes, foils or sheets with conical nanopores can be obtained by the known procedure of asymmetric chemical etching of the latent ion track as described in Nucl Instrum Methods Phys Res, Sect. B 2001, 184, 337-346. After heavy ion irradiation and subsequent chemical etching process of the polymer membranes, foils or sheets, carboxylic acid groups are generated on the surface of the polymer membrane, foil or sheet, in particular on the nanopore surface of the ion-track etched polymer membrane, foil or sheet, which can be used for covalent attachment of functional molecules containing for example a primary amine.

In a preferred embodiment, the sensor of the present application comprises a fluorophore and a polymer membrane with nanopores as detection means.

The sensor according to the present application may further comprise a linker moiety, which is preferably attached to the C-terminus of the amino acid or modified amino acid X. The linker moiety is for ensuring a spatial separation of the functional groups of the copper-binding motif and the fluorophore to the polymer membrane, foil or sheet as detection means, in particular ion-track etched PET-membrane having conical nanopores. The linker may also function to attach the sensor comprising the copper-binding motif with or without the fluorophore to a solid support, while ensuring a spatial separation of the functional groups of the copper-binding motif and the fluorophore to the surface of the solid support. Thereby an undesired interaction between the functional groups on the surface of the polymer membrane, foil or sheet or of the functional groups on the surface of the solid support and the copper-binding motif and fluorophore can be avoided.

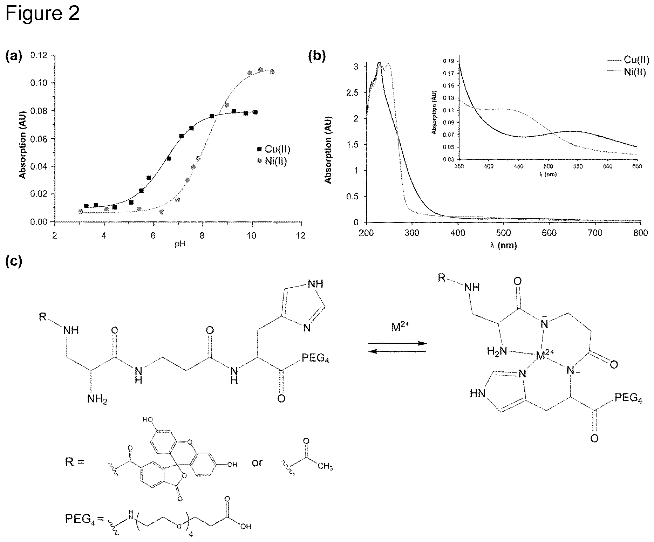

Preferably, the linker moiety comprises polyethylene glycol (PEG) units, in particular 4 PEG units. The linker moiety preferably has the formula R₁-[CH₂CH₂O]ₐ-R₂, wherein subscript a is from 2 to 10, R₁ is -NH and R₂ is CH₂CH₂COOH or CH₂CH₂CCH₃=O. In the formula R₁-[CH₂CH₂O]ₐ-R₂ a may be 2 to 8, 2 to 6, 2 to 4, 4 to 10, 4 to 8 or 4 to 6, more preferably a is 3, 4, 5 or 6 and most preferable a is 4. In a preferred embodiment the linker has the structure -HN-[CH₂CH₂O]₄-CH₂CH₂COOH (Figure 2(c)) referred to as linkerPEG₄ in the present invention. If the linker comprises a carboxy group R₂ = COOH coupling by means of said carboxy group to a polymer membrane, foil or sheet or to a solid support can be achieved. For example, the carboxy group of the linker may be coupled to an amino functional group on the surface of a polymer membrane or solid support via standard peptide coupling chemistry. The amino group R₁ allows coupling to the C-terminus of the amino acid or modified amino acid X, also by means of standard peptide coupling chemistry.

In one embodiment of the present application, the copper-binding motif with or without a fluorophore may be attached by means of the linker moiety to the surface of the polymer membrane with nanopores, in particular to the ion-track etched polymer membrane, foil or sheet, in particular to the surface of the nanopores. Binding of Cu²⁺ ions to the copper-binding motif of the sensor changes the current-voltage characteristics of the polymer membrane, foil or sheet with the nanopores which can then be used for qualitative and quantitative detection of Cu²⁺ ions in the fluid.

In one embodiment, the sensor according to the present application comprises the copper-binding motif, detection means and a linker, wherein the detection means is a fluorophore, in particular 5(6)-carboxyfluorescein and/or a polymer membrane with nanopores, preferably an ion-track etched PET-membrane, foil or sheet with nanopores. The fluorophore is attached to the N-terminus of the DAP and the linker moiety is attached to the C-terminus of the amino acid or modified amino acid X. The copper-binding motif may be attached via the linker moiety to the surface of the polymer membrane, foil or sheet, in particular to the surface of the nanopores of the ion-track etched PET-membrane.

The sensor according to the present application may further comprise a solid support. In one preferred embodiment, the sensor comprises the copper-binding motif, a fluorophore as detection means and a solid support. The copper-binding motif bound to the fluorophore may be immobilized on the solid support, which can be with or without the linker moiety, wherein immobilization or attachment by means of the linker moiety is preferred. When the linker moiety is employed, a spatial separation of the functional groups of the copper-binding motif and of the fluorophore to the surface of the solid support is achieved whereby an undesired interaction between the functional groups on the surface of the solid support and the copper-binding motif and fluorophore can be avoided.

The linker moiety may be attached to the copper-binding motif via the C-terminus of the amino acid or modified amino acid X to ensure a spatial separation of the functional groups of the fluorophore-copper-binding motif to the functional groups on the surface of the solid support. In a preferred embodiment, the sensor for detection of Cu²⁺ ions in a fluid comprises fluorophore-DAP-βAla-X-linkerPEG₄ attached to or immobilized on a solid support, wherein the fluorophore is preferably 5(6)-carboxyfluorescein and X is histidine, alanine or aspartic acid. The solid support may be for example a polymer, a resin, a glass substrate, a core-shell particle or cellulose. The solid support may be in the form of a membrane, foil, sheet, strip or particle.

The sensor according to the present application may be used for detecting Cu²⁺ ions in a fluid, which fluid can be obtained from any source. The fluid may comprise a liquid, preferably an aqueous or water-based liquid, for example from environmental sources. Examples for a liquid according to the present invention are drinking water and body fluids such as urine, whole blood, plasma, serum, extracellular interstitial fluid, lymph, bile, cerebrospinal liquid as well as gland secretion such as saliva or sweat, wherein urine is preferred. The aqueous or water-based liquid further may originate from water streams, puddles, lakes and seas.

The sensor according to the present application is able to selectively detect Cu²⁺ ions in a fluid in the presence of Ni²⁺ ions and/or Zn²⁺ ions. Selective detection of Cu²⁺ ions according to the present application means that in case no Cu²⁺ ions are present in the fluid, but Ni²⁺ ions and/or Zn²⁺ ions are, no signal indicating the presence of the desired analyte Cu²⁺ ions is generated by the detection means. For example, when using 5(6)-carboxyfluorescein as fluorophore, no quenching in fluorescence intensity would be observed. Further, the presence of Ni²⁺ ions and/or Zn²⁺ ions in addition to a particular concentration of Cu²⁺ ions in the fluid does not change the signal of the detection means by more than ± 5%, i.e. almost identical results are obtained for the Cu²⁺ ion concentration in a solution containing Ni²⁺ ions and/or Zn²⁺ ions as for a solution containing Cu²⁺ ions, but no Ni²⁺ ions and/or Zn²⁺ ions.

In one embodiment, the sensor according to the present application is for use in the diagnosis and/or monitoring of diseases, preferably of diseases linked to abnormal Cu²⁺ ion (copper) concentrations and/or dysregulated Cu²⁺ ion (copper) metabolism such as Alzheimer's disease, Wilson's disease and Menke's disease. Cu²⁺ ion concentrations in terms of diagnosis and/or monitoring of diseases, in particular when determining the Cu²⁺ ion concentration in urine refer to copper, i.e. Cu²⁺ ions that is not bound to ceruloplasmin (non-CP Cu). Such copper may also be termed "free Cu²⁺ ions" or "free Cu²⁺ ions in solution".

The term "abnormal" in relation to Cu²⁺ ion (copper) concentration according to the present application means that the Cu²⁺ ion concentration in a body fluid is higher or lower compared to healthy individuals. Higher Cu²⁺ ion concentration in a body fluid, for example urine, is observed in individuals having a particular disease such as Alzheimer's disease or Wilsons's disease. Lower Cu²⁺ ion concentration in a body fluid is observed in individuals having a disorder correlated to copper deficiency such as Menke's disease. In healthy individuals the concentration of non-ceruloplasmin-bound Cu²⁺ (non-CP Cu) in urine is in a range of 40 to 50 nM calculated based on a Cu²⁺ ion amount of about 4.82 µg/day in 1.5 to 2.0 L urine (J Trace Elem Met Biol 2018, 45, 181-188). Thus, a Cu²⁺ ion concentration in urine above 40-50 nM, for example at least twice as high than in healthy individuals, i.e. above 80 or 90 nM, in particular in a range of 100 to 130 nM as is often the case for AD patients is considered an abnormal copper or Cu²⁺ ion concentration.

In a preferred embodiment, the sensor according to the present application is for use in the diagnosis and/or monitoring of Alzheimer's disease (AD), Wilson's disease and Menke's disease. In a further preferred embodiment, the sensor according to the present application is for use in determining Cu²⁺ ion concentration in the urine of individuals to diagnose and/or monitor diseases linked to abnormal Cu²⁺ ion (copper) concentrations and/or dysregulated Cu²⁺ ion (copper) metabolism, in particular Alzheimer's disease, Wilson's disease and Menke's disease. The use of the sensor of the present application is in particular for an *ex vivo* determination of Cu²⁺ ion concentration in body fluids, preferably in the urine of individuals.

Alternatively, the sensor according to the present application may be for use in detecting Cu²⁺ ions and/or determining Cu²⁺ ion concentration in food products or fluids such as aqueous or water-based liquids, in particular in food/water control and/or environmental samples.

Particularly, the sensor according to the present application may be used for multiple measurements, i.e. the sensor allows a repeated measurement of Cu²⁺ ions or Cu²⁺ ion concentration.

The present application further discloses a method for the qualitative and quantitative detection of Cu²⁺ ions in a fluid. The method comprises the steps of adjusting the pH of a fluid sample to be analyzed to 5.0 to 8.5, preferably to a pH of 5.5. to 8.0, 6.0 to 7.3, 6.3 to 8.0 or 6.5 to 7.5 and most preferably to a pH of 6.5 to 7.0. By adjusting the pH of the fluid sample to be analyzed the selectivity of the sensor according to the present application towards Cu²⁺ ions is ensured, in particular selectivity towards Cu²⁺ ions over Ni²⁺ and Zn²⁺ ions. Setting the pH of the fluid sample to the claimed range, i.e. to a pH range of 5.0 to 8.5 leads to formation of a complex only between Cu²⁺ ions and the copper-binding motif of the sensor. The formation of Ni²⁺- or Zn²⁺-complexes at the claimed pH-range with the copper-binding motif of the sensor is insignificant.

Without being bound by theory, it is assumed that upon binding of the copper-binding motif, in particular DAP-βAla-His of the sensor to the Cu²⁺ ions a square planar complex is formed having a *d-d* transition band at 537 nm (Figure 2(c)). As can be seen in Figure 2(a) of the present application Ni-complexation of the sensor according to the present application significantly starts at around > pH 7, which Ni-complex shows a transition band at 438 nm (Figure 2(b)). Thus, adjusting the pH in relation to the pH-dependent formation of the Cu²⁺- and Ni²⁺-complex, taking into account up to which pH range the Ni²⁺-complex is not formed, a highly selective detection method for Cu²⁺ ions in a fluid is enabled.

Performing quantitative and/or qualitative detection of Cu²⁺ ions with the sensor according to the present application in a fluid with a pH range of 5.0 to 8.5, preferably in a pH range of 5.5. to 8.0, 6.0 to 7.3, 6.3 to 8.0 or 6.5 to 7.5 and even more preferably at a pH range of 6.5 to 7.0 corresponding to pH₅₀ of the copper-sensor, i.e. the pH value at which 50% of the Cu²⁺-complexes have been formed, allows for selective detection of Cu²⁺ ions also in the presence of Ni²⁺ ions in the fluid sample, since at the claimed pH range only insignificant or no complexation of Ni²⁺ is present.

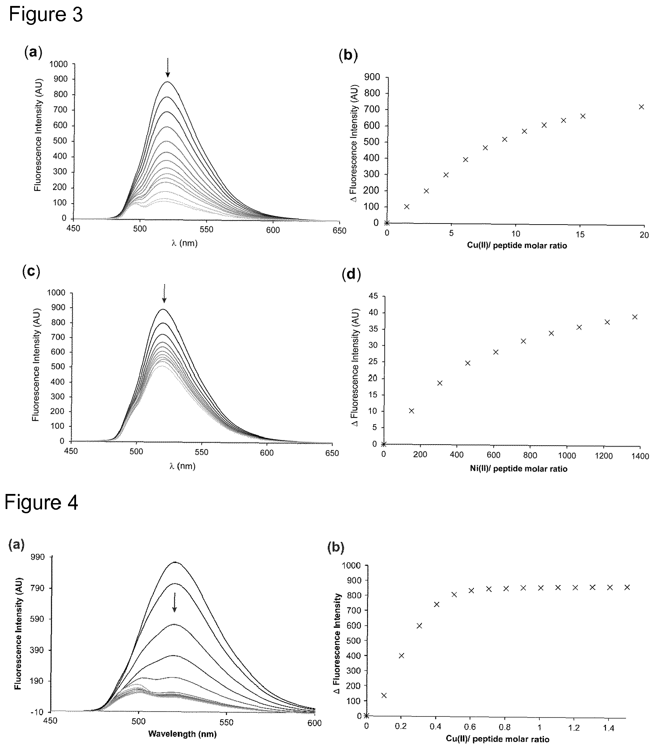

In a further step, following adjustment of the pH of the fluid sample, the sensor is brought into contact with the fluid sample. This may be done for example by immersing a test strip with the immobilized sensor in the fluid sample or by mixing a solution of the sensor of the present application with the fluid sample. In the next step, detection of Cu²⁺ ions and/or determining the Cu²⁺ ion concentration of the sample takes place. Depending on the detection means different parameters are used as basis for qualitative and/or quantitative detection of Cu²⁺ ions in the fluid sample. In case a sensor comprising a fluorophore as detection means, in particular 5(6)-carboxyfluorescein is used, quenching of the fluorescence intensity upon contact of the sensor with the analyte Cu²⁺ ions is observed. As can be seen in Figure 3(a),(b) a linear decrease of fluorescence intensity of the sensor upon addition of Cu²⁺ ions in water can be clearly observed showing more and more quenching of the fluorescence intensity with increasing Cu²⁺ ion concentrations.

When using a fluorophore as the detection means in the sensor according to the present application, the change in the fluorescence intensity is measured. Upon binding of the Cu²⁺ ions to the copper-binding motif of the sensor, the fluorescence intensity may be reduced, i.e. quenched. Cu²⁺ ion concentrations in the fluid sample with the sensor according to the present invention can be quantitatively detected in a range from 1 nM to 30 mM, 5 nM to 20 mM, 10 nM to 10 mM, 12 nM to 5 mM, in particular in a range from 1 nM to 60 µM, 5 nM to 40 µM, 10 nM to 10 µM, 12 nM to 5 µM, 1 nM to 500 nM, 1 nM to 200 nM or 12 nM to 150 nM. The quantitative detection of Cu²⁺ ion concentrations in a fluid sample as low as about 1 nM is very important for application of the sensor in the detection of trace amounts of copper ions in food/water samples or for detection of copper ions in the early diagnosis of AD in urine. A copper value, i.e. amount of Cu²⁺ ions of about 12 µg/day in the 24 h urine of AD patients (J Trace Elem Met Biol 2018, 45, 181-188) and taking into account that 24 h collected urine is around 1.5 to 2.0 L leads to a copper concentration in urine in a range of 100 to 130 nM for AD patients. The sensor according to the present application can be used to measure the copper (Cu²⁺) ion concentration directly from the urine sample in a simple two-step procedure, for example by immersing a test strip with the immobilized sensor in the urine sample or by mixing a solution of the sensor of the present application with the urine sample and then subjecting the sample to a fluorescence light source. For individuals with Wilson's disease the concentration of free Cu²⁺ ions in urine (Cu²⁺ ions not bound to ceruloplasmin (non-CP Cu)) is determined to be about 5 to 10 µM, which is even higher than in AD patients. For healthy individuals with a copper value of 4.82 µg/day the Cu²⁺ ion or copper concentration varies between 40 to 50 nM. All Cu²⁺ ion or copper values for healthy individuals and patients of AD and Wilson's disease are within the range of detection of the sensor according to the present application.

In an alternative embodiment, the qualitative and quantitative detection of Cu²⁺ ions in the fluid sample may be achieved by monitoring the change in the current-voltage (*I-V*) characteristics of the sensor comprising at least a copper-binding motif, a polymer membrane, foil or sheet with nanopores as detection means, a linker moiety wherein the copper-binding motif is attached via the linker moiety to the surface of the polymer membrane, foil or sheet with nanopores and optionally a fluorophore. Preferably, the polymer membrane, foil or sheet with nanopores is a PET membrane, foil or sheet with nanopores and even more preferably, the polymer membrane, foil or sheet with nanopores is an ion-track etched PET-membrane, foil or sheet with nanopores. In a preferred embodiment, the ion-track etched PET-membrane, foil or sheet has conical nanopores with a tip opening of 30 ± 5 nm and a big opening of 500 ± 10 nm in diameter. The polymer membrane, foil or sheet preferably contains about 10⁴ nanopores per cm². Optionally, the sensor may also include a fluorophore as detection means. By measuring the changes in the current-voltage (*I-V*) characteristics of the sensor, a quantitative and qualitative detection of Cu²⁺ ions in a fluid sample is feasible. In particular the detection of Cu²⁺ ion concentrations in a fluid sample with Cu²⁺ ion concentrations as low as 1 fM can be done, in particular in a range from 1 fM to 30 mM, 1 fM to 20 mM, 1 nM to 10 mM, 1 nM to 5 mM, in particular in a range from 1 fM to 60 µM, 5 nM to 40 µM, 10 nM to 10 µM, 12 nM to 5 µM, 1nM to 500 nM, 1 nM to 200 nM or 12 nM to 150 nM.

A qualitative and/or quantitative detection of Cu²⁺ ions in a fluid sample may also be performed with mass spectrometry analysis. A sensor according to the present application may then comprise only the copper-binding motif and no further detection means are necessary.

The sensor according to the present application may be regenerated to be applicable for further detection cycles. Regeneration is conducted by subjecting the sensor to a complexing agent. The complexing agent can be, for example, ethylene diamine tetraacetic acid (EDTA). In a preferred embodiment, the sensor according to the present application can be regenerated at least 6 times.

The sensor of the present application may be manufactured as follows. The copper-binding motif DAP-βAla-X may be synthesized following the standard solid phase peptide synthesis (SPPS). In case a fluorophore is used as detection means, the fluorophore may also be attached via standard peptide coupling chemistry to the N-terminus of the DAP. In case the sensor comprises a linker moiety said linker moiety may also be attached via standard peptide coupling chemistry to the C-terminus of amino acid or modified amino acid X. In case the sensor comprises a copper-binding motif, a linker moiety, a polymer membrane with nanopores as detection means and optionally a fluorophore as detection means, the copper-binding motif may be attached via the linker moiety to the surface of the polymer membrane with nanopores.

The sensor according to the present application can also be used in combination with a test strip for the detection of Cu²⁺ ions in a fluid sample. The test strip may comprise the sensor according to the present application immobilized via the linker moiety to a solid support, for example a polymer foil or sheet. Optionally, the sensor immobilized on the solid support may be applied to a carrier material. The solid support and/or the carrier material may have at least two slots or indentions into which a fluid sample may be applied. In one of the slots/indentions the fluid sample to be analyzed for the qualitative and/or quantitative detection of Cu²⁺ ions can be applied. In the further slot/indention a control solution may be applied. In case a quantitative detection of Cu²⁺ ions in the fluid sample should be performed in a further slot/indention a calibration solution may be applied. In a preferred embodiment, the sensor used in combination with a test strip comprises the copper-binding motif, a fluorophore as detection means, a linker moiety and a solid support. In that case, the calibration solution is for calibration of the fluorescence intensity.

The copper-binding motif 2,3-diaminopropionic acid(DAP)-βAla-X may be used for binding of Cu²⁺ ions in a fluid, wherein X is an amino acid or a modified amino acid, in particular wherein X is histidine (His), alanine (Ala) or aspartic acid (Asp). The copper-binding motif comprises in particular DAP-βAla-His or DAP-βAla-Asp.

### Description of Figures

**Figure 1** shows the "on-off" characteristics of a detection method using the sensor of the present application comprising 5(6)-carboxyfluorescein as detection means.
**Figure 2(a)** is an UV-vis spectrum showing the pH dependent formation of the Cu²⁺ complex (black square) at 537 nm of the sensor comprising Ac-DAP-βAla-His-linkerPEG₄ (1 mM, water) in relation to the formation of the corresponding Ni²⁺ complex at 438 nm of the sensor (grey dots). Boltzman fit (black and grey curves) show the sigmoidal trend of the pH titration curves. **Figure 2(b)** shows UV-vis spectra of the Cu²⁺ complex at pH 8 (black line) and Ni²⁺ complex at pH 10.5 (grey line) of the sensor comprising Ac-DAP-βAla-His-linkerPEG₄, the insert showing a magnification at 350 to 650 nm. **Figure 2(c)** is a scheme of the complex between the copper-binding motif DAP-βAla-His and the metal M²⁺ (Cu²⁺), wherein R is either 5(6)-carboxyfluorescein or acetyl.
**Figure 3(a)** is a fluorescence spectrum showing the decrease of fluorescence intensity of the sensor comprising 5(6)-carboxyfluorescein-DAP-βAla-His-linkerPEG₄ (1 µM, 2-(N-morpholino)ethanesulfonic acid (MES) buffer, pH 6.50) upon addition of CuSO₄ from 0 to 25 µM in water. **Figure 3(b)** shows the change in fluorescence intensity as a function of the molar ratio of Cu²⁺ to sensor (sensor is termed "peptide" in the description of the x-axis). **Figure 3(c)** is a fluorescence spectrum showing the decrease of fluorescence intensity of the sensor comprising 5(6)-carboxyfluorescein-DAP-βAla-His-linkerPEG₄ (1 µM, MES buffer, pH 6.50) upon addition of NiSO₄ from 0 to 3 mM in water. **Figure 3(d)** shows the change in fluorescence intensity as a function of the molar ratio of Ni²⁺ to sensor (sensor is termed "peptide" in the description of the x-axis).
**Figure 4(a)** is a fluorescence spectrum showing the decrease of fluorescence intensity of the sensor comprising 5(6)-carboxyfluorescein-DAP-βAla-His-linkerPEG₄ (0.1 µM, phosphate buffer, pH 8.0) upon addition of CuCI2 from 0 to 0.15 µM in water. **Figure 4(b)** shows the change in fluorescence intensity as a function of the molar ratio of Cu²⁺ to sensor (sensor is termed "peptide" in the description of the x-axis).

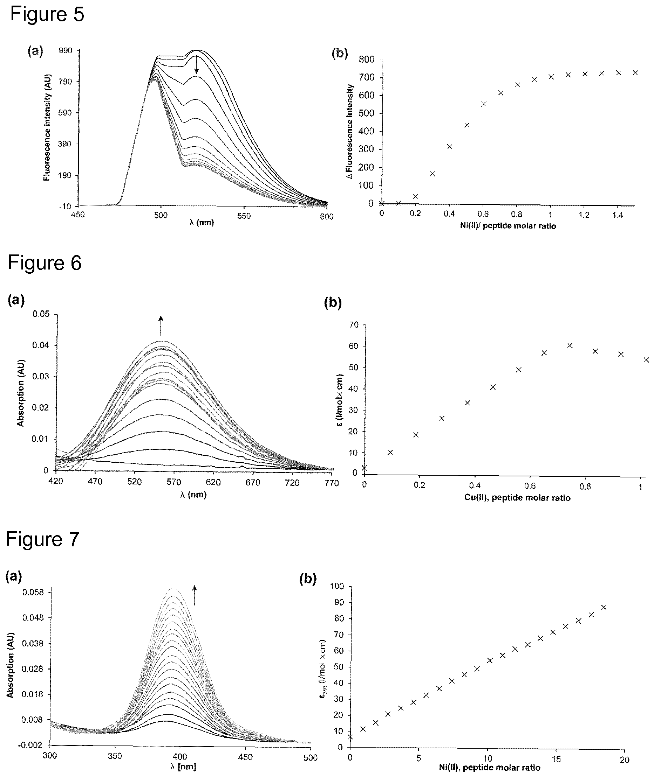

**Figure 5(a)** is a fluorescence spectrum showing the decrease of fluorescence intensity of the sensor comprising 5(6)-carboxyfluorescein-DAP-βAla-His-linkerPEG₄ (0.025 µM, CAPS buffer, pH 10.55) upon addition of Ni²⁺ from 0 to 0.038 µM in water. **Figure 5(b)** shows the change in fluorescence intensity as a function of the molar ratio of Ni²⁺ to sensor (sensor is termed "peptide" in the description of the x-axis).
**Figure 6(a)** is an UV-Vis titration spectrum of the sensor comprising Ac-DAP-βAla-His-linkerPEG₄ (0.68 mM, MES buffer pH 6.50) upon addition of Cu²⁺ (0 to 0.7 mM) in water. **Figure 6(b)** shows the absorption coefficient ε of the sensor-Cu²⁺ complex as a function of the molar ratio of Cu²⁺ to sensor (sensor is termed "peptide" in the description of the x-axis).
**Figure 7(a)** is an UV-Vis titration spectrum of the sensor comprising Ac-DAP-βAla-His-linkerPEG₄ (0.68 mM, MES buffer pH 6.50) upon addition of NiSO₄ (0 to 16 mM) in water. **Figure 7(b)** shows the change in absorption as a function of the molar ratio of Ni²⁺ to sensor (sensor is termed "peptide" in the description of the x-axis).

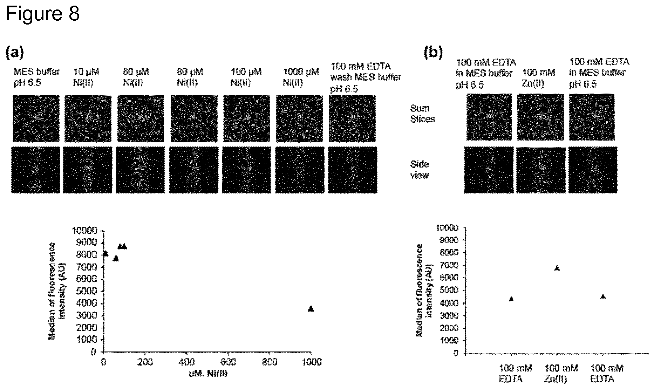

**Figure 8(a)** shows fluorescence emission images obtained with confocal laser scanning microscopy (CLSM) after addition of different amounts of NiSO₄ (top). It also shows the median of fluorescence intensity in relation to different amounts of NiSO₄ (bottom). **Figure 8(b)** shows a fluorescence image obtained with CLSM after addition of 100 mM ZnSO₄ (top). It also shows the median of fluorescence intensity after addition of 100 mM ZnSO₄ in relation to reference "washed out" nanopores fluorescence intensities with 100 mM EDTA (bottom).

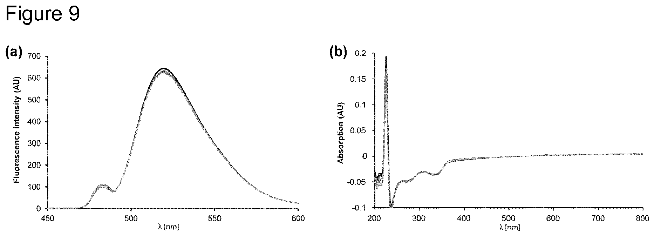

**Figure 9(a)** is a fluorescence spectrum showing fluorescence titration results of 5(6)-carboxyfluorescein-DAP-βAla-His-linkerPEG₄ (1 µM, 100 mM MES buffer, pH 6.50) upon addition of ZnSO₄ from 0 to 3 mM in water. **Figure 9(b)** shows an UV-vis titration spectrum of the sensor comprising Ac-DAP-βAla-His-linkerPEG₄ (1 mM, 100 mM MES buffer pH 6.50) upon addition of ZnSO₄ (0 to 2 mM) in water.

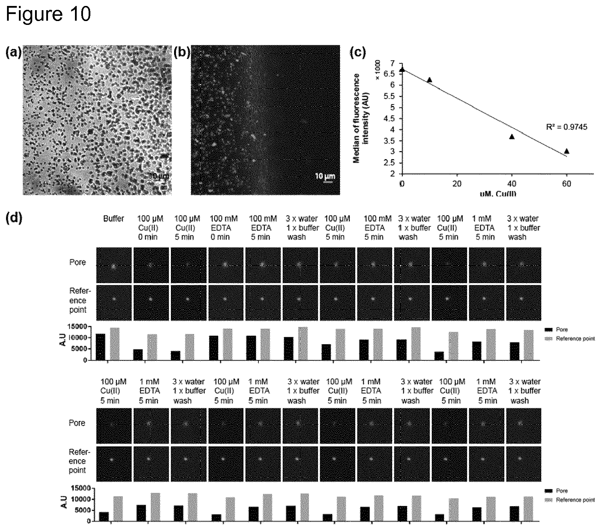

**Figure 10(a)** shows the surface of an ion-track etched PET-membrane with nanopores upon which 5(6)-carboxyfluorescein-DAP-βAla-His-linkerPEG₄ was immobilized (via linkerPEG₄ as the linker moiety) (reflected light). **Figure 10(b)** shows confocal laser scanning microscopy (CLSM) images of a sensor comprising 5(6)-carboxyfluorescein-DAP-βAla-His-linkerPEG₄ linked (via linkerPEG₄) to an ion-track etched PET-membrane with nanopores. The area on the right hand side represents nanopores which have not been etched and thus no 5(6)-carboxyfluorescein-DAP-βAla-His-linkerPEG₄ was immobilized thereon. **Figure 10(c)** shows the linear dependency of decrease in median fluorescence intensity with increase in Cu²⁺ ion concentration. **Figure 10(d)** shows CLSM fluorescence emission images of the "on-off" characteristics the sensor, in particular the re-usability of the sensor after regeneration with 1 mM EDTA and washing with water and MES buffer, pH 6.50.

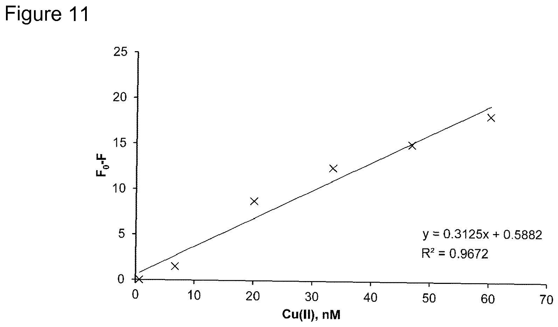

**Figure 11** shows a plot of F₀ - F in relation to Cu²⁺ ion concentration for determining the limit of detection (LOD) of the sensor comprising 5(6)-carboxyfluorescein-DAP-βAla-His-linkerPEG₄ (1 µM, 100 mM MES buffer pH 6.50; addition of CuSO₄ (0 to 60 nM) in water), wherein F₀ is the fluorescence intensity of the solution without analyte, i.e. without Cu²⁺ ions and F is the fluorescence intensity of the solution with Cu²⁺ ions.

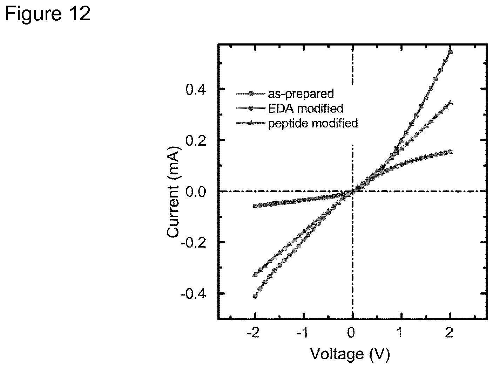

**Figure 12** shows the current-voltage (*I-V*) characteristics of the ion-track etched PET-membrane with nanopores without any sensor immobilized thereon (as-prepared), the ion-track etched PET-membrane with nanopores modified with EDA (EDA modified) and the ion-track etched PET-membrane with nanopores upon which 5(6)-carboxyfluorescein-DAP-βAla-His-linkerPEG₄ was immobilized (peptide modified).

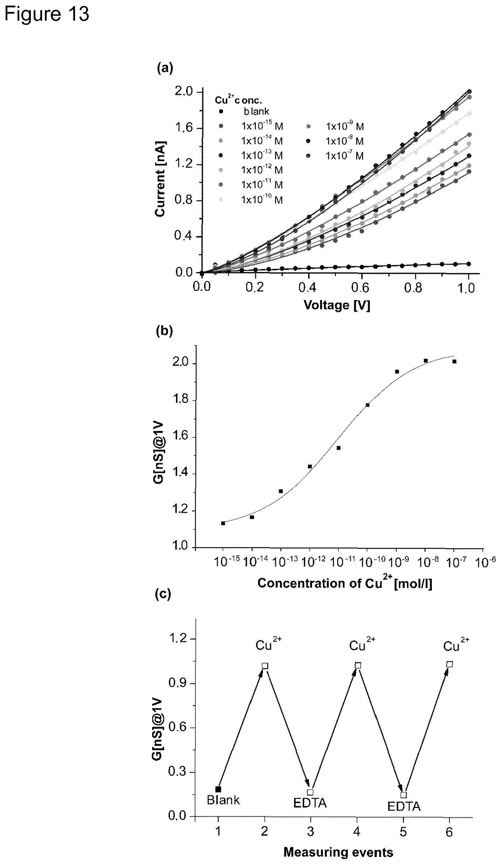

**Figure 13(a)** shows the current-voltage (*I-V*) characteristics of the sensor comprising the ion-track etched PET-membrane with nanopores upon which 5(6)-carboxyfluorescein-DAP-βAla-His-linkerPEG₄ was immobilized upon application of different concentrations of Cu²⁺ ions. **Figure 13(b)** shows the conductance depending on the Cu²⁺ ion concentration. **Figure 13(c)** shows the reversible complexation/decomplexation behavior of Cu²⁺ ions when using EDTAas complexing agent.

### Examples

### Solid phase peptide synthesis (SPPS) and characterization of copper-binding motifs

5(6)-carboxyfluorescein-DAP-βAla-His-linkerPEG₄ (**1**) and Acetyl(Ac)-DAP-βAla-His-linkerPEG₄ (**2**) were synthesized following the standard Fmoc-SPPS using chlorotrityl chloride resin (0.966 mmol/g). After linkerPEG₄ coupling the resin was capped with 8.5:1:0.5 dichloromethane (DCM)/methanol/N-ethyl-N-(propan-2-yl)propan-2-amine (DIEA) followed by coupling of Fmoc-His(Trt)-OH, Fmoc-βAla-OH, Fmoc-Dap(Boc) and 5(6)-carboxyfluorescein (fluorophore only for (**1**)), correspondingly. Deprotection was obtained by 20 % piperidine in N,N-dimethylformamide (DMF) and the coupling efficiency was monitored by UV-Vis-spectrometry at 301 nm. Sensor (**2**) was acetylated at the N-terminus with acetic anhydride. The final cleavage of sensors (**1**) and (**2**) from the resin was carried out in 2/2/48/48 triisopropylsilane(TIPS)/water/trifluoroacetic acid(TFA)/DCM and agitated for 1 h at room temperature.

RP-HPLC purification was performed on a C18 column (MultoKrom 100-5. 250 x 20 mm, 100 Å pore diameter, 5.0 µm particle size) using a linear gradient of 5 % to 40 % of eluent B (eluent A: water (0.1 % TFA) and eluent B: acetonitrile (0.1 % TFA)) in 60 min. Sensor (2) was purified following an isocratic method of 5 % eluent B for 12 minutes followed by a linear gradient to 30 % eluent B within a total of 60 minutes. The molecular mass was confirmed by ESI-MS ((**1**): m/z: [M+H]⁺ calculated for C44H51N7O15 918.92, found 918.35; (**2**): m/z: [M+H]⁺ calculated for C25H43N7O10 602.31, found 602.31).

Collected fractions were combined, freeze-dried and stored at -28°C. Purity of the collected fractions was confirmed by analytical RP-HPLC on a Waters XC e2695 system (Waters, Milford, MA, USA) employing a Waters PDA 2998 diode array detector equipped with ISAspher 100-3 C18 (C18, 3.0 µm particle size, 100 Å pore size, 50x4.6 mm, Isera GmbH, Düren, Germany). The sensor was eluted with a gradient of 0 % - 30 % eluent B in 10 min at a flow rate of 2 mL/min. Chromatograms were extracted at 214 nm. The molecular weight of the purified sensors as well as complexation of sensor (1) and (2) with Cu(II), Ni(ll) was confirmed by ESI mass spectrometry on a TOF-Q impact II spectrometer (Bruker Daltonik GmbH, Bremen, Germany) and calibrated using Bruker's ESI-Tune-Mix.

### Preparation of a sensor comprising the copper-binding motif, a fluorophore, a linker moiety and an ion-track etched PET-membrane with nanopores

Ion-track etched PET-membranes with conical nanopores were fabricated through asymmetric chemical etching of latent ion tracks as has been described in Nucl Instrum Methods Phys Res, Sect B 2001, 184, 337-346. PET foils or membranes were first irradiated with single swift heavy ions (Au) of kinetic energy 11.4 MeV/nucleon at the linear accelerator UNILAC (GSI Helmholtz Centre for Heavy Ion Research, Darmstadt, Germany). Then, the latent ion tracks in the polymer foils or membranes were sensitized with soft UV light. The chemical track-etching process was performed in a conductivity cell. The ion tracked foils or membranes were fixed in between two chambers of the cell. An etching solution (9 M NaOH) was filled in one chamber and a stopping solution (1 M KCI + 1 M HCOOH) was filled in the other chamber. The etching process was carried out at room temperature. The etching process was monitored by applying a potential of -1 V across the foil or membrane. The etching process was stopped when the current reached a certain defined value after the breakthrough point. Then, the etched foil or membrane was washed with stopping solution and dipped in deionized water overnight to remove residual salts.

The carboxylic acid groups on the polymer foil or membrane surface, in particular on the surface of the nanopores originated from the chemical etching. These groups were first activated through standard carbodiimide coupling chemistry. The track-etched foil or membrane was exposed to an ethanol solution containing 1-ethyl-3-(3-dimethylaminopropyl)carbodiimide (EDC) (100 mM) and pentafluorophenyl (PFP) (200 mM) at room temperature for 1 h. After washing with ethanol several times, the activated polymer foil or membrane was treated with ethylenediamine (EDA, 50 mM) solution overnight. During this reaction period, amine-reactive PFP-esters were covalently coupled with amine group of the EDA. Subsequently, the modified polymer foil or membrane was washed thoroughly with ethanol followed by careful rinsing with deionized water.

Then, the EDA-modified foil was used for the immobilization of the sensor having carboxylic acid groups at the terminus of the linker moiety (Figure 2(c)). Carboxylic acid groups of the linker were activated with 1-[bis(dimethylamino)methylene]-1H-1,2,3-triazolo[4,5]pyridinium-3-oxide hexa-fluorophosphate (HATU)/DIEA and reacted with sensor (1) or (2) (0.5 mM) in DMF, and left to react overnight at room temperature.

### Characterization of the Cu²⁺ and Ni²⁺ complex with the copper-binding motif of the sensor and selectivity of the sensor of the present application for Cu²⁺ ions over Ni²⁺ and Zn²⁺ ions

Fluorescence studies performed with sensor (**1**) at pH 8.0 at which pH optimal formation of the Cu-sensor-complex was observed, show that when adding one equivalent of Cu²⁺ ions, fluorescence intensity is quenched to 89 % (11 % remained intensity) by formation of a non-fluorescent complex between the Cu²⁺ ions and the copper binding motif (Figures 4(a),(b)). For Ni²⁺ ions pH 10.55 was evaluated as the lowest pH at which complete formation of the Ni-complex was achieved, and upon addition of 1 equivalent Ni²⁺ ions a remaining fluorescence of 29 % is observed (Figures 5(a),(b)). To obtain a distinguished selectivity of the sensor towards Cu²⁺ ions, a pH of 6.50 was determined for copper-binding motif DAP-βAla-His. This can be seen in the pH titration study shown in Figure 2(a). pH Titration studies were performed using a 1 mM sensor solution in deionized water. One equivalent of the according metal ion, Cu²⁺ or Ni²⁺, was added and the pH lowered to pH < 3 using 0.1 mM HCI. Adding small aliquots of 0.1 mM NaOH the pH was increased and with each step a spectrum recorded.

At pH of 6.50 nickel ion complexation is insignificant whereas for copper this pH represents the pH₅₀. UV-Vis titration using different Cu²⁺ ion concentrations shows the anticipated behavior at pH₅₀ illustrating a saturation after addition of around 0.5 equivalents Cu²⁺ ions (Figures 6(a),(b)).

The fluorescence spectrum in Figure 3(a) shows the decrease of fluorescence intensity of the sensor comprising 5(6)-carboxyfluorescein-DAP-βAla-His-linkerPEG₄ (1) (1 µM, MES buffer, pH 6.50) upon addition of CuSO₄ from 0 to 25 µM. Emission (A) was determined to be 518 nm. Fluorescence titration upon addition of 20 equivalents of Cu²⁺ at pH 6.50 showed quenching of the fluorescence intensity to 88% (12% remaining fluorescence intensity) (Figure 3(b)). In comparison thereto, fluorescence titration upon addition of nickel at pH 6.50 showed quenching of the fluorescence intensity after addition of 1364 equivalents of nickel to only 43% (57% remaining fluorescence intensity) (Figures 3(c),(d)) which can be explained by the insufficient formation of the sensor-nickel complex at pH 6.50.

Further, an experiment with Ni²⁺ and the sensor comprising Ac-DAP-βAla-His-linkerPEG₄ (2) at a pH of 6.50 did not show the required maximum absorption at 438 nm which indicates the formation of the planar coordination of Ni²⁺ to the sensor, but rather showed a maximum absorption at 394 nm indicating that the complex between the copper-binding motif and the Ni²⁺ ions of the sensor is not assembled but rather a non-specific octahedral binding is achieved (Figures 7(a),(b)). Thus, it was confirmed that when adjusting the pH to the claimed range, a selectivity of the sensor according to the present application is guaranteed for Cu²⁺ over Ni²⁺ and the almost exclusive formation of the copper-binding complex is confirmed.

In Figure 8(a) it is demonstrated that there is no decrease in fluorescence intensity visible for Ni²⁺ ions in a range of 0 to 200 µM in MES buffer, pH 6.50. Only upon addition of 1 mM (1000 µM) NiSO₄ in MES buffer a reduction in fluorescence intensity is observed. With regard to Zn (Figure 8(b)) it can be seen that even addition of 100 mM ZnSO₄ in MES buffer, pH 6.50 does not affect the fluorescence intensity. The titration experiments were performed at pH 6.50 to demonstrate selectivity towards Cu²⁺ ions.

The binding constants for Cu²⁺ and Ni²⁺ to the copper-binding motif of the sensor of the present application were determined by UV-Vis spectroscopy at pH 6.50 using the Benesi-Hildebrand method. The obtained binding constants were similar to the binding constants determined with fluorescence spectroscopy using the Stern-Volmer plot at pH 6.50. Binding constants of the sensor are as follows log*K_{Cu}* = 6.8 for 5(6)-carboxyfluorescein-DAP-βAla-His-linkerPEG₄ (**1**) with K_{b} = 6.27 × 10⁶ M⁻¹ and log*K_{Cu}* = 6.56 for Ac-DAP-βAla-His-linkerPEG₄ (**2**) with K_{b} = 4.00 × 10⁶ M⁻¹ showing a slightly better binding to the sensor comprising the fluorophore. At pH 6.50 the binding constants towards nickel were determined to be log*K_{Ni}* = 3.26 with K_{b} = 1.80 × 10³ M⁻¹ for (1) and log*K_{Ni}* = 2.76 with with K_{b} = 5.80 × 10² M⁻¹ for (**2**) demonstrating again the outstanding selectivity towards copper. By optimizing the pH value a superior copper binding of the copper-binding motif of the sensor of the present application was achieved, leading to a selective formation of the Cu-complex at the respective pH value.

No binding of Zn²⁺ ions was detected for 5(6)-carboxyfluorescein-DAP-βAla-His-linkerPEG₄ (1) or Ac-DAP-βAla-His-linkerPEG4 (2) (Figures 9(a),(b)).

### Measurement of fluorescence intensity of the sensor comprising the copper-binding motif, 5(6)-carboxyfluorescein as detection means, a linker moiety and an ion-track etched PET-membrane with nanopores

When using fluorescence intensity as parameter for determining the presence and/or concentration of Cu²⁺ ions, in particular when using 5(6)-carboxyfluorescein as fluorophore a decrease in fluorescence intensity indicates the presence of Cu²⁺ ions. The higher the Cu²⁺ concentration the lower the fluorescence intensity. This has been shown in Figures 10(a)-(d). Figures 10(a)-(d) show 5(6)-carboxyfluorescein-DAP-βAla-His-linkerPEG₄ (1) attached to an ion-track etched PET-membrane with nanopores (via the linker moiety linkerPEG₄) which was subjected to confocal laser scanning microscopy (CLSM) measurements.

5(6)-carboxyfluorescein-DAP-βAla-His-linkerPEG₄ (1) is almost exclusively present on the surface of the nanopores of the ion-track etched PET-membrane or foil as can be seen in Figure 10(a) and displays a green color when excited by the laser at 488 nm in the CLSM image (Figure 10(b)). Figure 10(d) shows a titration experiment showing the dependency of decrease in fluorescence with the increase in Cu²⁺ ion concentration as well as the re-usability of the sensor of the present application. CuSO₄ was added in concentrations from 0 to 100 µM in MES buffer solution, pH 6.50. In particular, the "on-off" characteristics of the sensor of the present application showing re-usebility after regeneration with 1 mM EDTA and washing with water and MES buffer, pH 6.50 can be seen in said figure. Figure 10(c) shows clearly the linear dependency of decrease in fluorescence with the increase in Cu²⁺ ion concentration. This experiment was performed by measuring the decrease in median fluorescence intensity after addition of CuSO₄ from 0 to 60 µM in MES buffer, pH 6.5.

As can be seen in Figures 10(c) and (d) fluorescence intensity decreases with addition of Cu²⁺ with a linear dependency. Furthermore, when EDTA was used as complexing agent it was possible to regenerate the Cu²⁺ sensitivity of the sensor. As can be seen in Figure 10(d) the sensing towards Cu²⁺ could be revived for at least seven times. Further, a shelf life of the sensor of at least six months was confirmed.

Also the limit of detection (LOD) for Cu²⁺ ions using the fluorescence signal was determined. In order to determine the sensitivity of the sensor according to the present application the limit of detection of the sensor comprising 5(6)-carboxyfluorescein-DAP-βAla-His-linkerPEG₄ (1) towards Cu²⁺ ions in solution from fluorescence intensity titration studies is defined to be 12 nM. This value is extremely important for application of the sensor according to the present invention for detection of trace amounts of copper ions in food/water control or detection of copper ions for the early diagnosis of Alzheimer's disease in urine.

The LOD was calculated using the following equation: LOD = (3 × σ)/slope, wherein σ = standard deviation of blank solution. 1 µL of a blank solution (100 mM MES buffer pH 6.50) was pipetted to a 1 µM peptide solution in 100 mM MES buffer and the difference between fluorescence intensity F₀-F relating to the starting intensity was determined (Figure 11). From a six fold titration study the standard deviation of the change in fluorescence intensity with the addition of blank solution is determined and used as the σ value (1.28) (Eur J Biochem 2002, 269, 1323-1331)

### Measurement of the current-voltage (I-V) characteristics of the sensor comprising the copper-binding motif, a fluorophore, a linker moiety and an ion-track etched PET-membrane with nanopores as detection means

In the following the measurement of the current-voltage (*I-V*) characteristics of the sensor according to the present application is outlined. The sensor comprises the copper-binding motif, a fluorophore and a linker moiety, i.e. 5(6)-carboxyfluorescein-DAP-βAla-His-linkerPEG₄ whereby the copper-binding motif with the fluorophore is attached to the ion-track etched PET-membrane with nanopores by means of the linker. The PET-membrane with nanopores behaves as an ohmic resistor before Cu²⁺ complexation meaning that the net surface charge on the nanopore surface is zero and no flux from the narrow cone opening to the wide opening takes place. The ion-track etched PET-membrane with conical nanopores having carboxyl groups on the surface with no immobilized copper-binding motif (as-prepared) shows a flux from the narrow cone opening to the wide opening because of the negative carboxyl groups (Figure 12). After modification with ethylene diamine (EDA) the surface charge switches from negative to positive resulting in an anion selectivity and the concomitant inversion of the rectification behavior of the nanopore (EDA modified). Immobilization of the copper-binding motif with the fluorophore via the linker to the surface of the nanopores of the ion-track etched PET-membrane results in change of the nanopore transport behavior from a rectifying to a non-rectifying due to the loss of nanopore surface charges. The PET-membrane with the immobilized copper-binding motif behaves as an ohmic resistor, i.e. the net surface charge on the nanopore walls is zero.

Upon complexation of Cu²⁺ by the sensor, i.e. by the copper-binding motif, a positive charge is generated on the nanopore surface, which concomitantly changes the ion transport behavior of the nanopore. The nanopore becomes ion selective as evidenced from the successive increase in positive current by increasing the Cu²⁺ concentration ranging from fM to nM concentrations as shown in Figures 13(a) and (b). Thus, the presence of the generated Cu complex on the surface of the nanopores switches the nanopore transport behavior from a non-conducting "off" state to a conducting "on" state. As can be further seen in Figures 13(a) and (b) the sensor according to the present application is able to recognize Cu²⁺ ions even at concentrations as low as 1 fM and this recognition process can be transduced in an electronic signal originating from the transport behavior of the nanopore. From the data given in Figure 13 it can be seen that the sensor exhibits a remarkable interaction ability towards Cu²⁺ due to the particular copper-binding motif used in the sensor according to the present application.

The reproducibility of the conductance measurements over at least six cycles is shown in Figure 13(c).

The present application further relates to the following items.
Item 1. A sensor for detection of Cu²⁺ ions in a fluid comprising a copper-binding motif 2,3-diaminopropionic acid(DAP)-βAla-X, wherein X is an amino acid or a modified amino acid, in particular wherein X is histidine (His), alanine (Ala) or aspartic acid (Asp).
Item 2. The sensor according to item 1 comprising the copper-binding motif 2,3-diaminopropionic acid(DAP)-βAla-Hisor DAP-βAla-Asp, wherein DAP is preferably L-DAP.
Item 3. The sensor according to items 1 or 2 further comprising detection means and/or a linker moiety.
Item 4. The sensor according to item 3, wherein the detection means comprises a fluorophore, in particular 5(6)-carboxyfluorescein.
Item 5. The sensor according to item 3 or 4, wherein the detection means is attached to the N-terminus of the DAP.
Item 6. The sensor according to any one of items 3 to 5 comprising the copper-binding motif DAP-βAla-X, wherein X is an amino acid or a modified amino acid, in particular wherein X is histidine (His), alanine (Ala) or aspartic acid (Asp) and DAP is L-DAP, and a fluorophore, wherein the fluorophore is preferably attached to the N-terminus of the DAP.
Item 7. The sensor according to item 3, wherein the linker moiety is attached to the C-terminus of the X.
Item 8. The sensor according to any one of items 3 to 7 comprising the copper-binding motif DAP-βAla-X, wherein X is an amino acid or a modified amino acid, in particular wherein X is histidine (His), alanine (Ala) or aspartic acid (Asp), a fluorophore, wherein the fluorophore is preferably attached to the N-terminus of the DAP, and a linker moiety, wherein the linker moiety is preferably attached to the C-terminus of the amino acid or a modified amino acid X.
Item 9. The sensor according to any one of items 3 to 8, wherein the detection means comprises a polymer membrane with nanopores, preferably a functionalized polymer membrane with nanopores and more preferably an ion-track etched PET membrane with nanopores.
Item 10. The sensor according to any one of items 3 to 9 comprising the copper-binding motif DAP-βAla-X, wherein X is an amino acid or a modified amino acid, in particular wherein X is histidine (His), alanine (Ala) or aspartic acid (Asp), a linker moiety, wherein the linker moiety is preferably attached to the C-terminus of the amino acid or a modified amino acid X, and a polymer membrane with nanopores, preferably a functionalized polymer membrane with nanopores and more preferably an ion-track etched PET membrane with nanopores and optionally a fluorophore, wherein the fluorophore is preferably attached to the N-terminus of the DAP.
Item 11. The sensor according to item 10, wherein a functionalized polymer membrane with nanopores is a membrane with functional groups such as carboxylate and/or amino groups on the surface of the membrane, in particular on the surface of the nanopores.
Item 12. The sensor according to any one of items 9 to 11, wherein the copper-binding motif, optionally with a fluorophore, is attached via the linker moiety to the surface of the polymer membrane, in particular to the surface of the nanopores.
Item 13. The sensor according to any one of items 3 to 12, wherein the linker moiety comprises polyethylene glycol, in particular wherein the linker moiety has the formula R₁-[CH₂CH₂O]ₐ-R₂, wherein a is preferably 4, R₁ is preferably -NH and/or R₂ is preferably COOH, wherein a linker moiety having the structure -HN-[CH₂CH₂O]₄-CH₂CH₂COOH (linkerPEG₄) or -HN-[CH₂CH₂O]₄-CH₂CH₂COO⁻ is most preferred.
Item 14. The sensor according to any one of items 3 to 13 comprising 5(6)-carboxyfluorescein-DAP-βAla-His-linkerPEG₄ or 5(6)-carboxyfluorescein-DAP-βAla-Asp-linkerPEG₄.
Item 15. The sensor according to any one of items 1-14 further comprising a solid support.
Item 16. The sensor according to item 15, wherein the copper-binding motif optionally with a fluorophore is attached via the linker moiety to the solid support, in particular wherein the linker moiety comprises polyethylene glycol, in particular wherein the linker moiety has the structure -HN-[CH₂CH₂O]₄-CH₂CH₂COO⁻.
Item 17. The sensor according to items 15 or 16 comprising the copper-binding motif DAP-βAla-X, wherein X is an amino acid or a modified amino acid, in particular wherein X is histidine (His), alanine (Ala) or aspartic acid (Asp) and a fluorophore, wherein the fluorophore is preferably attached to the N-terminus of the DAP, and wherein the copper-binding motif with the fluorophore is attached to the solid support via the linker moiety.
Item 18. The sensor according to any one of items 15 to 17, wherein the solid support is a polymer, a resin, a glass substrate, a core-shell particle or cellulose and/or wherein the solid support is in the form of a membrane, foil, sheet, strip or particle.
Item 19. The sensor according to any one of items 1-18 for selectively detecting Cu²⁺ ions in a fluid in the presence of Ni²⁺ ions and/or Zn²⁺ ions.
Item 20. The sensor according to any one of items 1-19 for use in the diagnosis and/or monitoring of diseases, preferably of diseases linked to abnormal Cu²⁺ ion concentrations and/or dysregulated Cu²⁺ ion metabolism, in particular Alzheimer's disease, Wilson's disease and Menke's disease.
Item 21. The sensor according to any one of items 1-19 for use in determining Cu²⁺ ion concentration in the urine of individuals to diagnose and/or monitor diseases linked to abnormal Cu²⁺ ion concentrations and/or dysregulated Cu²⁺ ion metabolism, in particular Alzheimer's disease, Wilson's disease and Menke's disease.
Item 22. The sensor according to any one of items 1-19 for use in determining Cu²⁺ ion concentration ex vivo in the urine of individuals to diagnose and/or monitor diseases linked to abnormal Cu²⁺ ion concentrations and/or dysregulated Cu²⁺ ion metabolism, in particular Alzheimer's disease, Wilson's disease and Menke's disease.
Item 23. The sensor according to any one of items 20-22 for use, wherein an abnormal Cu²⁺ ion concentration relates to a Cu²⁺ ion concentration in a body fluid such as urine and/or serum which is higher or lower than in a healthy individual.
Item 24. The sensor according to any one of items 1-19 for use in detecting Cu²⁺ ions and/or determining Cu²⁺ ion concentration in food products or fluids, in particular in environmental water samples.
Item 25. A method for detection of Cu²⁺ ions and/or for determining Cu²⁺ ion concentration in a fluid sample comprising the steps of
   adjusting the pH of a fluid sample to be analyzed to 5.0 to 8.5, in particular to a pH of 6.3 to 7.0,
   contacting the sensor according to any one of items 1-19 with the fluid sample, and
   determining the presence and/or concentration of Cu²⁺ ions in the fluid sample by measuring a change in the parameter of the detection means, and
   optionally further comprising a step of regenerating the sensor by subjecting the sensor to a complexing agent.
Item 26. The method according to item 25, wherein the complexing agent comprises EDTA.
Item 27. The method according to item 25, wherein the change in fluorescence intensity is measured, and/or wherein the Cu²⁺ ion concentration in the fluid sample is in a range of 1 nM to 60 µM, in particular in a range of 1 nM to 200 nM.
Item 28. The method according to item 18, wherein the change in the current-voltage characteristics is measured, and/or wherein the Cu²⁺ ion concentration in the fluid sample is in a range of 1 fM to 60 µM, in particular in a range of 1 fM to 200 nM.
Item 29. Test strip for the detection of Cu²⁺ ions in a fluid sample comprising the sensor according to any one of items 1-19.
Item 30. Use of a copper-binding motif 2,3-diaminopropionic acid(DAP)-βAla-X for binding of Cu²⁺ ions in a fluid, wherein X is an amino acid or a modified amino acid, in particular wherein X is histidine (His), alanine (Ala) or aspartic acid (Asp) and/or DAP is L-DAP.
Item 31. Use of the copper-binding motif according to item 30, wherein the copper binding motif comprises 2,3-diaminopropionic acid(DAP)-βAla-His or 2,3-diaminopropionic acid(DAP)-βAla-Asp, wherein DAP is preferably L-DAP.
Item 32. A method for manufacturing the peptide sensor according to items 3-19 comprising the steps of
   synthesizing a compound including the Cu-binding motif DAP-βAla-X, the linker moiety and the fluorophore according to items 1 to 19 by solid phase peptide synthesis,
   providing a polymer membrane, foil or sheet, and
   coupling the compound via the linker moiety to the surface of the polymer membrane.

## Claims

1. A sensor for detection of Cu²⁺ ions in a fluid comprising a copper-binding motif 2,3-diaminopropionic acid(DAP)-βAla-X, wherein X is an amino acid or a modified amino acid, in particular wherein X is histidine (His), alanine (Ala) or aspartic acid (Asp).

2. The sensor according to claim 1 further comprising detection means and/or a linker moiety.

3. The sensor according to claim 2, wherein the detection means comprises a fluorophore, in particular 5(6)-carboxyfluorescein.

4. The sensor according to claims 2 or 3, wherein the detection means comprises a polymer membrane with nanopores, preferably an ion-track etched PET membrane with nanopores.

5. The sensor according to any one of claims 2-4, wherein the linker moiety comprises polyethylene glycol, in particular wherein the linker moiety has the formula R₁-[CH₂CH₂O]ₐ-R₂, wherein a is preferably from 2 to 10, R₁ is preferably -NH and R₂ is preferably CH₂CH₂COOH.

6. The sensor according to any one of claims 1-3 and 5 further comprising a solid support.

7. The sensor according to any one of claims 1-6 for selectively detecting Cu²⁺ ions in a fluid in the presence of Ni²⁺ ions and/or Zn²⁺ ions.

8. The sensor according to any one of claims 1-7 for use in the diagnosis and/or monitoring of diseases, preferably of diseases linked to abnormal Cu²⁺ ion concentrations and/or dysregulated Cu²⁺ ion metabolism, in particular Alzheimer's disease, Wilson's disease and Menke's disease.

9. The sensor according to any one of claims 1-7 for use in determining Cu²⁺ ion concentration in the urine of individuals to diagnose and/or monitor Alzheimer's disease.

10. The sensor according to any one of claims 1-7 for use in detecting Cu²⁺ ions and/or determining Cu²⁺ ion concentration in food products or fluids, in particular in environmental water samples.

11. A method for detection of Cu²⁺ ions and/or for determining Cu²⁺ ion concentration in a fluid sample comprising the steps of
adjusting the pH of a fluid sample to be analyzed to 5.0 to 8.5, in particular to a pH of 6.3 to 7.0,
contacting the sensor according to any one of claims 1-7 with the fluid sample, and
determining the presence and/or concentration of Cu²⁺ ions in the fluid sample by measuring a change in the parameter of the detection means, and
optionally further comprising a step of regenerating the sensor by subjecting the sensor to a complexing agent.

12. The method according to claim 11, wherein the change in fluorescence intensity is measured, and/or wherein the Cu²⁺ ion concentration in the fluid sample is in a range of 1 nM to 60 µM, in particular in a range of 1 nM to 200 nM.

13. The method according to claim 11, wherein the change in the current-voltage characteristics is measured, and/or wherein the Cu²⁺ ion concentration in the fluid sample is in a range of 1 fM to 60 µM, in particular in a range of 1 fM to 200 nM.

14. Test strip for the detection of Cu²⁺ ions in a fluid sample comprising the sensor according to any one of claims 1-7.

15. Use of a copper-binding motif 2,3-diaminopropionic acid(DAP)-βAla-X for binding of Cu²⁺ ions in a fluid, wherein X is an amino acid or a modified amino acid, in particular wherein X is histidine (His), alanine (Ala) or aspartic acid (Asp).
